Europäisches Patentamt

European Patent Office

Office européen des brevets

(19)

(11) **EP 0 682 250 B1**

(12) **FASCICULE DE BREVET EUROPEEN**

(45) Date de publication et mention
de la délivrance du brevet:
**12.06.2002 Bulletin 2002/24**

(51) Int Cl.$^7$: **G01N 33/18**, G01N 21/33

(21) Numéro de dépôt: **95400985.8**

(22) Date de dépôt: **28.04.1995**

(54) **Procédé et dispositif pour la caractérisation des matières organiques, azotées et en suspension dans un liquide**

Verfahren und Vorrichtung zur Kennzeichnung der organischen, stickstoffenthaltenden und suspendierten Materialien in einer Flüssigkeit

Method and device for characterising organic matter, nitrogen containing matter and suspended matter in a liquid

(84) Etats contractants désignés:
**DE GB IT**

(30) Priorité: **13.05.1994 FR 9406037**

(43) Date de publication de la demande:
**15.11.1995 Bulletin 1995/46**

(73) Titulaire: **SECOMAM S.A.**
**95334 Domont Cédex (FR)**

(72) Inventeurs:
- **Thomas, Olivier**
  **F-73000 Barberaz (FR)**
- **Rigal, Jean-Marc**
  **F-95330 Domont (FR)**
- **Constant, Daniel**
  **F-95880 Enghien les Bains (FR)**

(74) Mandataire: **de Saint-Palais, Arnaud Marie**
**CABINET MOUTARD**
**35 Rue de la Paroisse**
**BP 513**
**78005 Versailles Cedex (FR)**

(56) Documents cités:
DE-A- 3 223 167          US-A- 4 244 696
US-A- 5 298 428

- **ADVANCES IN INSTRUMENTATION PART I, vol.34, 1 Octobre 1979 pages 333 - 341 KUCZYNSKI 'APPLICATIONS OF TOC MEASUREMENTS'**
- **PATENT ABSTRACTS OF JAPAN vol. 12, no. 95 (P-681) (2942) 29 Mars 1988 & JP-A-62 228 145 (SHIMADZU) 7 Octobre 1987**
- **TM TECHNISCHES MESSEN, vol.61, no.5, 1 Mai 1994 pages 204 - 207 RINKE ET AL. 'MEHRKOMPONENTE PROZESS UV SPEKTROMETER'**
- **PATENT ABSTRACTS OF JAPAN vol. 12, no. 95 (P-681) (2942) 29 Mars 1988 & JP-A-62 228 146 (SHIMADZU) 7 Octobre 1987**
- **ATP AUTOMATISIERUNGSTECHNISCHE PRAXIS, vol.34, no.3, 1 Mars 1992 pages 128 - 136 RAAB ET AL. 'KONTINUIERLICHE TOC MESSTECHNIK,ETC.'**
- **PATENT ABSTRACTS OF JAPAN vol. 6, no. 34 (P-104) 2 Mars 1982 & JP-A-56 153 238 (FUJI ELECTRIC) 27 Novembre 1981**

EP 0 682 250 B1

**Description**

La présente invention concerne un procédé et un dispositif pour la caractérisation des matières organiques, azotées et en suspension dans un liquide.

Elle s'applique plus particulièrement, mais non exclusivement, au contrôle en continu de la qualité des eaux ainsi qu'au contrôle du niveau de traitement des effluents (rendement d'épuration et qualité du rejet) dans les stations d'épuration.

D'une manière générale, on sait que depuis près d'un siècle, la pollution oxydable liée à la présence de matières organiques est évaluée par des mesures de demande en oxygène (DCO ou DBO). De nos jours, ces mesures tendent à être remplacées par d'autres méthodes plus rapides, moins polluantes, plus précises ou plus significatives comme la mesure du carbone organique. Malheureusement, les solutions existantes sont complexes et onéreuses et les différentes tentatives de mesure indirectes se sont révélées inefficaces.

Par ailleurs, la recherche de solutions analytiques simples a conduit au développement de systèmes optiques permettant une mesure rapide et directe sans traitement de l'échantillon ni ajout de réactif, les différents paramètres concernés par les mesures optiques étant, en premier lieu, les paramètres globaux comme la turbidité, les matières en suspension et les matières organiques, mais aussi des constituants minéraux (nitrates, certains métaux...) ou organiques (phénols, détergents...).

Ainsi, notamment, le résumé Patent Abstract of Japan, Vol 12, n° 95 (P. 681) (2942) du 29 mars 1988 & JP-A-62 228 146 (SHIMADZU) décrit un procédé de mesure UV d'une substance organique dans lequel des erreurs sont évitées en irradiant l'échantillon avec des rayons ultraviolets et en calculant la réduction d'absorbance avant et après la décomposition par oxydation. Toutefois, ce procédé ne permet pas de caractériser plusieurs paramètres significatifs de la présence de matières organiques dans un échantillon.

La publication Advances in Instrumentation, vol. 34, part 1 du 1er octobre 1979, pages 333-341 met en relief l'effet retardateur des matières en suspension dans des réactions d'oxydation de matières organiques.

L'invention a plus particulièrement pour objet un procédé et un dispositif permettant d'effectuer la susdite caractérisation par absorptiométrie ultraviolette, méthode qui consiste à exploiter le spectre d'absorption de l'eau pour en déduire les valeurs de certains paramètres utiles en vue de déterminer les concentrations des éléments ou composés recherchés et d'autres données physico-chimiques caractéristiques de l'échantillon.

Ainsi, par exemple dans le but d'estimer la concentration en carbone organique ou les matières en suspension, l'invention propose de déterminer à l'aide d'un spectrophotomètre, la variation de l'absorbance dans une plage de longueurs d'ondes comprises entre 200 et 300 nanomètres.

Dans ce cas, la relation entre le signal UV et le carbone organique (paramètre le plus significatif de la présence des matières organiques), s'appuie sur la relation de Beer-Lambert dont la propriété d'additivité permet de dire que l'absorbance mesurée, dans le cas d'un mélange en solution dilué, est égale à la somme des absorbances de chaque constituant dans la solution. Ces absorbances sont fonction de la concentration en carbone organique puisque cette dernière est liée à la concentration du constituant.

Cependant, selon la nature des molécules organiques prédominantes dans l'échantillon, qui varie avec le type d'effluent, la relation entre le carbone organique et le spectre sera différente puisqu'une même quantité de carbone peut être associée à un nombre différent de chromophores.

Pour tenir compte de ce facteur, l'invention se base sur la constatation que le spectre d'un échantillon inconnu peut être considéré comme une combinaison linéaire d'un petit nombre de spectres correspondant à des situations de référence parfaitement caractérisées. Ces spectres de référence SR qui sont caractéristiques des états de la matière organique, permettent de définir tout spectre inconnu SI sous la forme d'une combinaison linéaire :

$$SI = a_1\ SR_1 + a_2\ SR_2 + ... + a_p\ SR_p$$

où $a_1$ ... $a_p$ sont les coefficients des p spectres de référence $SR_1$ ... $SR_p$.

La connaissance des spectres de référence et de leurs coefficients, et des caractéristiques physico-chimiques des échantillons dont ils proviennent, permet de calculer n'importe quel paramètre de l'échantillon inconnu (en supposant que ce paramètre soit additif). En particulier, la concentration en carbone organique $CO_i$ d'un échantillon inconnu est calculée à l'aide de la même combinaison linéaire, appliquée cependant aux concentrations respectives de carbone organique $CO_1$ ... $CO_p$ des spectres caractéristiques :

$$CO_i = a_1\ .\ CO_1 + a_2\ .\ CO_2 + ... + a_p\ .\ CO_p$$

où $CO_i$, $CO_1$, ... $CO_p$ sont les valeurs respectives de carbone organique.

La méthode suppose donc connu les spectres caractéristiques (sélectionnés par exemple à partir de plusieurs centaines d'échantillons) et la valeur de carbone organique qui leur est associée.

L'invention a plus particulièrement pour but un procédé et un dispositif qui permettent d'exploiter une méthode de ce type, non seulement pour la détermination des concentrations en carbone organique, mais également des composés azotés sous toutes ses formes, présent dans un liquide tel que, par exemple, une eau naturelle ou une eau usée ou résiduaire.

Pour parvenir à ce résultat, l'invention propose un procédé comprenant la destruction partielle par action physique des matières en suspension dans le liquide, une phase de photo-oxydation destinée à réaliser une dégradation des matières organiques et une oxydation des composés azotés. Selon l'invention, ce procédé est caractérisé en ce qu'il comprend :

- la sélection d'une pluralité de spectres de référence obtenus à partir d'échantillons dont les susdits paramètres sont connus et la réalisation de tables de correspondance relatives auxdits spectres de référence et auxdits paramètres,

- la définition du spectre SI de l'échantillon inconnu sous la forme d'une combinaison linéaire de type :

$$SI = a_1 \ SR_1 + a_2 \ SR_2 + ... + a_p \ SR_p$$

formule dans laquelle $a_1 ... a_p$ sont des coefficients des spectres de référence $SR_1 ... SR_p$,

- le calcul, par déconvolution, desdits paramètres de l'échantillon inconnu, à l'aide d'une combinaison linéaire homologue de la précédente mais appliquée à chacun des paramètres, cette combinaison linéaire présentant, pour ce qui concerne la concentration en carbone organique $CO_i$ de l'échantillon inconnu, la formule suivante :

$$CO_i = a_1 CO_1 + a_2 CO_2 + ... + a_p CO_p$$

formule dans laquelle $CO_i$, $CO_1 ... CO_p$ sont les Valeurs respectives de carbone organique des échantillons de référence correspondant aux spectres $SR_1$, $SR_2 ... SR_p$.

Avantageusement, la destruction partielle des matières en suspension dans le liquide sera obtenue par broyage et/ou par dispersion ultrasonique, tandis que la phase de photo-oxydation est obtenue par exposition du liquide à un rayonnement ultraviolet.

Par ailleurs, le procédé selon l'invention pourra comprendre après la phase de destruction partielle des matières en suspension, une succession de cycles de photo-oxydation et de mesure spectrophotométrique, cette succession de cycles étant poursuivie jusqu'à ce que les mesures spectrophotométriques se stabilisent.

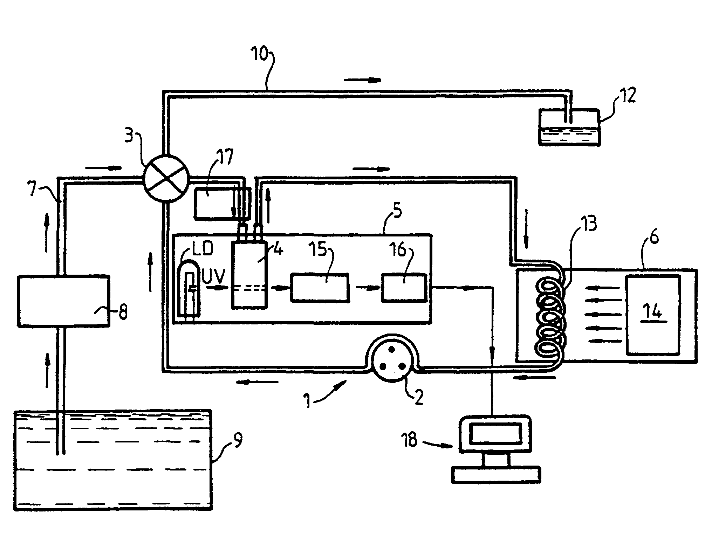

Un mode d'exécution d'une installation pour la mise en oeuvre du procédé selon l'invention sera décrit ci-après, à titre d'exemple non limitatif, avec référence au dessin annexé dans lequel :

La figure unique représente un schéma synoptique de l'installation.

Dans cet exemple, l'installation comprend un circuit de photo-oxydation en boucle fermée 1 comportant une pompe 2 débitant successivement dans une vanne quatre voies 3, dans une cuve à circulation 4 d'un système spectrophotométrique 5 et d'un poste d'exposition aux ultraviolets 6 permettant la dégradation des matières organiques et l'oxydation des composés azotés par photo-oxydation, ce poste d'exposition 6 étant relié par sa sortie à l'entrée de la pompe 2. La vanne quatre voies 3 est, quant à elle, reliée, d'une part, à un circuit d'admission 7 de liquide à analyser qui comprend un poste de destruction partielle des matières en suspension 8 dont l'entrée est connectée à une réserve 9 contenant les échantillons et, d'autre part, à un circuit de refoulement 10, dans un conduit d'évacuation 12, du liquide une fois analysé.

La pompe 2 utilisée peut consister en une pompe péristaltique de type classique. Elle sert à aspirer les échantillons de liquide traités par le poste de destruction 8, à faire circuler ces échantillons dans le circuit en boucle fermée 1 jusqu'à l'obtention de la stabilité des mesures, puis à évacuer les échantillons en fin de mesure dans le circuit d'évacuation 12.

Le poste de destruction 8 peut constituer en un broyeur dont le pot peut, par exemple, présenter une capacité de 100 ml environ. Le temps de broyage peut, par exemple, varier de 30 secondes à 3 minutes selon le type d'échantillon. Il peut être remplacé ou complété par un dispositif à ultrasons.

Le poste d'exposition 6 peut comprendre un serpentin 13 en matière transparente par exemple en quartz, exposé au rayonnement d'une source UV, 14. La circulation (en boucle fermée) de l'échantillon dans ce poste 6 s'effectue pendant tout le temps nécessaire pour obtenir l'oxydation quasi-complète des matières organiques et azotées. Ce résultat se manifeste par la stabilisation des mesures relevées par le système spectrophotométrique 5.

Le système spectrophotométrique 5 peut avantageusement comprendre un spectrophotomètre de type "S.750" fabriqué et commercialisé par la Demanderesse, comportant une lampe "deutérium" LD permettant l'émission d'un rayonnement UV au travers de la cuve de circulation 4, laquelle, réalisée en quartz, est placée dans la boucle de photo-oxydation 1 et un ensemble monochromateur 15-détecteur 16 destiné à recevoir le rayonnement UV ayant traversé la cuve de circulation 4. Cette cuve peut ménager un trajet optique variable en fonction des caractéristiques de l'échantillon.

Avantageusement, un dispositif simple de refroidissement 17 (maintien à température ambiante) de l'échantillon est placé en amont du système spectrophotométrique 5.

Les informations relatives aux spectres détectés par le détecteur 16 (par exemple entre 200 et 300 nm) sont transmises à un poste de traitement 18 pouvant comprendre un micro-ordinateur programmé de manière à assurer une déconvolution des spectres (avec prise en compte des interférences) pour la détermination du carbone organique, de l'azote nitreux et nitrique et des matières en suspension. Cette détermination peut être effectuée en utilisant des tables de correspondances préenregistrées relatives aux spectres de référence $SR_1$ ... $SR_p$ évoqués ainsi qu'aux caractéristiques physico-chimiques des échantillons dont ils proviennent.

Ce micro-ordinateur peut en outre effectuer un calcul des paramètres d'oxydabilité en mesurant par exemple le temps de stabilisation des mesures d'un échantillonnage circulant dans le circuit de photo-oxydation en boucle fermée.

Le fonctionnement de l'installation précédemment décrite est alors le suivant :

L'admission d'un nouvel échantillon dans le circuit de photo-oxydation 1 s'obtient en actionnant le vanne quatre voies 3 de manière à connecter le circuit 7 situé en aval du poste de destruction 8 au circuit situé en amont de la cuve à circulation 4 et le circuit de refoulement de la pompe 2 au circuit d'évacuation 10 des échantillons.

De ce fait, la pompe 2 aspire dans le circuit de photo-oxydation 1 un nouvel échantillon, tandis qu'elle refoule l'échantillon précédemment traité dans le conduit d'évacuation 12.

Lorsque le nouvel échantillon occupe la totalité du circuit de photo-oxydation 1, la vanne quatre voies 3 est actionnée de manière à reboucler le circuit de photo-oxydation 1 (connexion du circuit de refoulement de la pompe 2 à l'entrée de la cuve à circulation 4) et à obturer le circuit de sortie du poste de destruction 8 ainsi que le circuit d'évacuation 12.

Du fait de ce rebouclage, l'échantillon entraîné par la pompe 2 effectue une pluralité de cycles au cours de chacun desquels il passe successivement au poste de photo-oxydation 6 et dans la cuve à circulation 4 du système spectrophotométrique 5.

Le microprocesseur ordonne alors périodiquement des cycles de mesure qu'il compare aux précédents de manière à détecter la stabilisation de ces mesures (stabilisation qui correspond à l'oxydation quasi-complète des matières organiques et azotées). Pendant la procédure, l'ordinateur valide et effectue un traitement des valeurs relevées pour déterminer notamment la composition de l'échantillon en carbone organique, et en composés azotés.

Le microprocesseur peut ensuite provoquer l'actionnement de la vanne quatre voies 3 pour prélever un nouvel échantillon.

Les résultats obtenus au cours de la mesure peuvent concerner :

- la détermination quantitative de l'azote global, ammoniacal, nitreux et nitrique,
- l'estimation du carbone organique total et dissous,
- l'estimation des matières en suspension,
- l'estimation des paramètres de photo-oxydabilité de l'échantillon et, en particulier :

- . de la fraction oxydable, et donc du carbone oxydable et réfractaire à l'oxydation,
- . de la vitesse initiale d'oxydation,
- . du temps de demi-oxydation,
- . du temps de début de nitrification.

Grâce au procédé et à l'installation précédemment décrite, il devient possible d'améliorer considérablement le diagnostic du fonctionnement des systèmes d'assainissement et l'épuration, le contrôle de la qualité des rejets et la surveillance des eaux naturelles.

**Revendications**

1. Procédé pour la caractérisation par absorptiométrie ultraviolette de paramètres significatifs de la présence de carboné organique, de composés azotés et de matières en suspension dans un liquide, ce procédé comprenant **le broyage et/ou la dispersion ultrasonique** des matières en suspension dans le liquide, une phase de photo-oxydation destinée à réaliser une dégradation des matières organiques et une oxydation des composés azotés, **caractérisé en ce qu'**il comprend :

   - la sélection d'une pluralité de spectres de référence obtenus à partir d'échantillons dont les susdits paramètres sont connus et la réalisation de tables de correspondance relatives auxdits spectres de référence et auxdits paramètres,
   - la définition du spectre SI de l'échantillon inconnu sous la forme d'une combinaison linéaire de type :

$$SI = a_1\, SR_1 + a_2\, SR_2 + ... + a_p\, SR_p$$

   formule dans laquelle $a_1$ ... $a_p$ sont des coefficients des spectres de référence $SR1$ ... $SRp$,
   - le calcul, par déconvolution, desdits paramètres de l'échantillon inconnu, à l'aide d'une combinaison linéaire homologue de la précédente mais appliquée à chacun des paramètres, cette combinaison linéaire présentant, pour ce qui concerne la concentration en carbone organique $CO_i$ de l'échantillon inconnu, la formule suivante :

$$CO_i = a_1 CO_1 + a_2 CO_2 + ... + a_p CO_p$$

   formule dans laquelle $CO_1$ ... $CO_p$ sont les valeurs respectives de carbone organique des échantillons de référence correspondant aux

spectres $SR_1$, $SR_2$ ... $SR_p$.

**2.** Procédé selon la revendication 1,
**caractérisé en ce que** la susdite phase de photo-oxydation comprend l'exposition du liquide à un rayonnement ultraviolet.

**3.** Procédé selon l'une des revendications précédentes,
**caractérisé en ce qu'**après la phase de **broyage et/ou de dispersion ultrasonique,** l'échantillon à analyser comprend une succession de cycles de photo-oxydation et de mesures spectrophotométriques, cette succession de cycles étant poursuivie jusqu'à stabilisation des mesures spectrophotométriques.

**4.** Procédé selon l'une des revendications précédentes,
**caractérisé en ce que** le liquide entrant dans le poste de mesure spectrophométrique est maintenu à température ambiante.

**5.** Dispositif pour la caractérisation du carbone organique, des composés azotés et/ou des matières en suspension dans un liquide par spectrophotométrie UV, conformément au procédé selon l'une des revendications précédentes,
**caractérisé en ce qu'**il comprend un circuit de photo-oxydation en boucle fermée (1) comportant une pompe (2) débitant dans un circuit comprenant successivement des moyens de connexion (3), une cuve à circulation (4) d'un système spectrophotométrique (5) couplé à un micro-ordinateur et un poste d'exposition du liquide aux ultraviolets (6) dont la sortie est connectée à l'entrée de ladite pompe (2), le dispositif de connexion (3) étant connecté, d'une part, à la sortie d'un poste de destruction partielle par broyage et/ou par ultrasons (8) d'un circuit de prélèvement d'échantillons et, d'autre part, à un conduit d'évacuation de liquide (12), et **en ce que** le micro-ordinateur contient des tables de correspondance préenregistrées relatives à des spectres de référence $SR_1$ ... $SR_p$ connus d'une multiplicité d'échantillons et aux paramètres connus de ces spectres les plus significatifs de la présence en carbone organique, en composés azotés et en matière en suspension, ledit micro-ordinateur étant programmé pour définir le spectre SI de l'échantillon inconnu sous la forme d'une combinaison linéaire des spectres de référence $SR_1$ ... $SR_p$, respectivement associés à des coefficients $a_1$ ... $a_p$ et un calcul par déconvolution de chacun des paramètres de l'échantillon inconnu, à l'aide d'une combinaison linéaire des valeurs respectives de ce paramètre, dans les échantillons de référence en utilisant les susdits coefficients $a_1$ ... $a_p$.

**6.** Dispositif selon la revendication 5,
**caractérisé en ce que** le poste d'exposition (6) comprend un serpentin en matière transparente (13) exposé au rayonnement d'une source UV, (14).

**7.** Dispositif selon la revendication 5,
**caractérisé en ce que** le système spectrophotométrique (5) comprend une lampe (LD) permettant l'émission d'un rayonnement UV à travers une cuve de circulation (4) et un ensemble monochromateur-détecteur (15, 16) destiné à recevoir le rayonnement UV ayant traversé ladite cuve (4).

**8.** Dispositif selon la revendication 7,
**caractérisé en ce que** les informations relatives aux spectres détectés par le détecteur (16) sont transmises **au susdit micro-ordinateur (18) lequel est** programmé de manière à effectuer une déconvolution des spectres.

**Patentansprüche**

**1.** Verfahren für die Charakterisierung von aussagekräftigen Parametern für das Vorhandensein von organischem Kohlenstoff, Stickstoffverbindungen und Schwebstoffen in einer Flüssigkeit durch UV-Absorptionsmessung, wobei das Verfahren **die Zerkleinerung und/oder Ultraschalldispersion** der Schwebstoffe in der Flüssigkeit, eine Phase der Fotooxidation für die Herbeiführung eines Zerfalls der organischen Stoffe und eine Oxidation der stickstoffhaltigen Verbindungen beinhaltet,
**dadurch gekennzeichnet, dass** es folgendes beinhaltet:

- die Auswahl einer Mehrzahl von Referenzspektren, die anhand von Wasserproben erzielt worden sind, bei denen die oben genannten Parameter bekannt sind, und die Aufstellung von Vergleichstabellen in Bezug auf die genannten Referenzspektren und die genannten Parameter,

- die Definition des Spektrums SI der unbekannten Wasserprobe in Form einer linearen Kombination des Typs:

$$SI = a_1\, SR_1 + a_2\, SR_2 + ... a_p\, SR_p$$

wobei $a_1$ ... $a_p$ die Koeffizienten der Referenzspektren $SR_1$ ... $SR_p$ sind,

- die Berechnung der genannten Parameter der unbekannten Wasserprobe mittels Entfaltung mithilfe einer linearen Kombination, die der obigen entspricht, aber auf jeden der Parameter angewendet wird, wobei diese lineare Kombination im Hinblick auf die Konzentration der unbekannten Wasserprobe an organischem Kohlenstoff $CO_i$ die folgende Formel aufweist:

$$CO_i = a_1 \cdot CO_1 + a_2 \cdot CO_2 + ... + a_p \cdot CO_p$$

wobei $CO_i$, $CO_1$ ... $CO_p$ die jeweiligen Werte an organischem Kohlenstoff der Referenzwasserproben angeben, die den Spektren $SR_1$, $SR_2$ ... $SR_p$ entsprechen.

2. Verfahren gemäß Patentanspruch 1,
   **dadurch gekennzeichnet, dass** die oben genannte Phase der Fotooxidation die Aussetzung der Flüssigkeit gegenüber einer UV-Strahlung beinhaltet.

3. Verfahren gemäß einem der vorangegangenen Patentansprüche,
   **dadurch gekennzeichnet, dass** die zu analysierende Wasserprobe nach der Phase der <u>Zerkleinerung und/oder Ultraschalldispersion</u> eine Abfolge von Fotooxidationszyklen und spektrofotometrischen Messungen durchläuft, die fortgesetzt werden, bis sich die spektrofotometrischen Messungen stabilisiert haben.

4. Verfahren gemäß einem der vorangegangenen Patentansprüche,
   **dadurch gekennzeichnet, dass** die Flüssigkeit, die in die Station für die spektrofotometrische Messung gelangt, bei Raumtemperatur gehalten wird.

5. Vorrichtung für die Charakterisierung des organischen Kohlenstoffes, der stickstoffhaltigen Verbindungen und/oder der Schwebstoffe in einer Flüssigkeit mittels UV-Spektrofotometrie in Übereinstimmung mit dem Verfahren gemäß einem der voranstehenden Patentansprüche,
   **dadurch gekennzeichnet, dass** es einen geschlossenen Fotooxidationskreislauf (1) mit einer Pumpe (2) beinhaltet, die nacheinander in ein Vierwegeventil (3), in den Umlaufbehälter (4) eines spektrofotometrischen Systems (5) abgibt, gekoppelt mit einem Rechner und einer Station für die UV-Bestrahlung (6), deren Ausgang an den Eingang der Pumpe (2) angeschlossen ist. Das Verbindungsglied (3) wiederum ist einerseits an eine Station für die teilweise Zerstörung der Schwebstoffe (8) mittels Zerkleinerung und/oder Ultraschall eines Kreislaufs für die Entnahme von Wasserproben angeschlossen, und zum anderen an eine Ablaufleitung (12) für die Flüssigkeit; und dadurch dass der Rechner vorab aufgezeichnete Vergleichstabellen in Bezug auf bekannte Referenzspektren $SR_1$ ... $SR_p$ einer Vielzahl von Wasserproben und auf die bekannten Parameter dieser Spektren enthält, die hinsichtlich des Vorhandenseins von organischem Kohlenstoff, stickstoffhaltigen Verbindungen und Schwebstoffen am aussagekräftigsten Sind, wobei der genannte Rechner programmiert ist, um das Spektrum SI der unbekannten Wasserprobe in Form einer linearen Kombination der Referenzspektren $SR_1$ ... $SR_p$ zu definieren, die jeweils Koeffizienten $a_1$ ... $a_p$ zugeordnet werden, und um eine Berechnung mittels Entfaltung jedes der Parameter der unbekannten Wasserprobe mithilfe einer linearen Kombination der jeweiligen Werte dieser Parameter in den Referenzproben unter Verwendung der oben genannten Koeffizienten $a_1$ ... $a_p$ vorzunehmen.

6. Vorrichtung gemäß Patentanspruch 5,
   **dadurch gekennzeichnet, dass** die Station für die UV-Aussetzung (6) eine Rohrschlange aus einem transparenten Material (13) beinhaltet, die der Strahlung einer UV-Quelle (14) ausgesetzt wird.

7. Vorrichtung gemäß Patentanspruch 5,
   **dadurch gekennzeichnet, dass** das spektrofotometrische System (5) eine Lampe (LD) beinhaltet, die die Aussendung einer UV-Strahlung durch einen Umlaufbehälter (4) hindurch ermöglicht, und einen Monochromator-Sensor (15, 16) für die Aufnahme der UV-Strahlung, die den genannten Behälter (4) passiert hat.

8. Vorrichtung gemäß Patentanspruch 7,
   **dadurch gekennzeichnet, dass** die Informationen über die Spektren, die von dem Sensor (16) geortet worden sind, <u>an den oben genannten Computer (18) übermittelt werden,</u> der programmiert ist, um eine Entfaltung der Spektren vorzunehmen

**Claims**

1. Method for **characterising by** ultra-violet absorption of significant parameters of the presence of organic carbon, nitrogenous compounds and matter suspended in a liquid, said method comprising the mixing and/or ultrasonic dispersion of the matter suspended in the liquid, a photo-oxidation phase for degrading organic matter and oxidizing the nitrogenous compounds, said method being **characterised in that** it includes :

   - selecting a plurality of reference spectra obtained from samples whose said parameters are known and the drawing up of correspondence tables relating to said reference spectra and said parameters.
   - defining the spectrum SI of the unknown sample in the form of linear combination of the following type:

$$SI = a_1 \, SR_1 + a_2 \, SR_2 + ... + a_p \, SR_p$$

a formula in which $a_1...a_p$ are coefficients of the reference spectra SR1...SRp,
- calculating by "deconvolution" said parameters of the unknown sample with the aid of a homologous linear combination of the preceding one but applied to each parameter, said linear combination having, as regards the $CO_i$ organic carbon concentration of the unknown sample, the following formula :

$$CO_i = a_1 CO_1 + a_2 CO_2 + ... + a_p CO_p$$

a formula in which $CO_1$ - $CO_p$ are the respective organic carbon values of the reference samples corresponding to the spectra $SR_1$, $SR_2...SR_p$.

2. Method according to claim 1, **characterised in that** said photo-oxidation phase includes exposing the liquid to an ultraviolet radiation.

3. Method according to one of the preceding claims, **characterised in that** following the mixing and/or ultrasonic dispersion phase, the sample to be analysed includes a succession of photo-oxidation and spectrophotometric measurement cycles, said succession of cycles being carried out until the spectrophotometric measurements have stabilised.

4. Method according to one of the preceding claims, **characterised in that** the liquid entering the spectrophotometric measurement station is maintained at ambient temperature.

5. Device for characterising organic carbon, nitrogenous compounds and/or suspended matter in a liquid by means of ultraviolet spectrophotometry in accordance with the method according to one of the preceding claims, **characterised in that** it includes a closed loop photo-oxidation circuit (1) comprising a pump (2) discharging into a circuit successively including connection means (3), a circulation tank (4) of a spectrophotometric system (5) connected to a microcomputer and a station (6) for exposing the liquid to ultraviolet radiation, the outlet of said station being connected to the inlet of said pump (2), the connection device (3) being connected on the one hand to the outlet of a partial destruction station by mixing and/or ultrasounds (8) of a sampling circuit and, and on the other hand to a liquid evacuation channel (12), and **in that** the microcomputer contains pre-recorded correspondence tables relating to known $SR_1$ ... $SR_p$ reference spectra of a multiplicity of samples and the known parameters of the most significant spectra of the presence of organic carbon, nitrogenous compounds and suspended matter, said microcomputer being programmed so as to define the spectrum SI of the unknown sample in the form of a linear combination of the reference spectra $SR_1$ ... $SR_p$, respectively associated with the coefficients $a_1$ ... $a_p$ and a calculation via deconvolution of each parameter of the unknown sample with the aid of a linear combination of the respective values of this parameter in the reference samples by using said coefficients $a_1$... $a_p$.

6. Device according to claim 5, **characterised in that** the exposure station (6) comprises a serpentine (13) made of transparent material exposed to the radiation of a UV source (14).

7. Device according to claim 5, **characterised in that** the spectrophotometric system (5) comprises a lamp (LD) allowing the emission of an UV radiation through a circulation tank (4) and a monochromator-detector unit (15, 16) for receiving the UV radiation which has traversed said tank (4).

8. Device according to claim 7, **characterised in that** the information relating to the spectra detected by the detector (16) are transmitted to said microcomputer (18) which is programmed to carry out a deconvolution of the spectra.